(19) Europäisches Patentamt

European Patent Office

Office européen des brevets

(11) **EP 2 149 601 B1**

(12) **EUROPEAN PATENT SPECIFICATION**

(45) Date of publication and mention
of the grant of the patent:
**05.12.2012 Bulletin 2012/49**

(51) Int Cl.:
***C12N 5/07*** (2010.01)

(21) Application number: **08740506.4**

(22) Date of filing: **16.04.2008**

(86) International application number:
**PCT/JP2008/057427**

(87) International publication number:
**WO 2008/129997 (30.10.2008 Gazette 2008/44)**

(54) **COMPOSITION OF SOLUBILIZED AMNION AND USE THEREOF**

ZUSAMMENSETZUNG VON SOLUBILISIERTEM AMNION UND VERWENDUNG DAVON

COMPOSITION D'AMNIOS SOLUBILISÉE ET UTILISATION DE CETTE COMPOSITION

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **17.04.2007 JP 2007108077**

(43) Date of publication of application:
**03.02.2010 Bulletin 2010/05**

(73) Proprietor: **Nidek Co., Ltd.
Gamagori, Aichi 443-0038 (JP)**

(72) Inventors:
• **SAKURAGAWA, Norio
Kodaira-shi
Tokyo 187-0032 (JP)**
• **KAMO, Isao
Yokohama-shi
Kanagawa 225-0011 (JP)**

(74) Representative: **Kador & Partner
Corneliusstrasse 15
80469 München (DE)**

(56) References cited:
**WO-A2-2004/076631     WO-A2-2004/076631
JP-A- 2001 161 353**

• UCHINO Y ET AL: "Amniotic membrane- poly (vinyl alcohol) hybrid polymer as an artificial cornea scaffold" IOVS, vol. 46, no. Suppl. S, 2005, page 4986, XP9131292 & ANNUAL MEETING OF THE ASSOCIATION-FOR-RESEARCH-IN-VISION-AND-OPH THALMOLOGY; FT LAUDERDALE, FL, USA; MAY 01 -05, 2005 ISSN: 0146-0404
• KUMAR T R ET AL: "Biocompatible collagen scaffolds from a human amniotic membrane: physicochemical and in vitro culture characteristics" JOURNAL OF BIOMATERIALS SCIENCE. POLYMER EDITION, VSP, UTRECHT, NL, vol. 14, no. 7, 1 January 2003 (2003-01-01), pages 689-706, XP009117131 ISSN: 0920-5063
• KOBAYASHI M. ET AL.: 'Enhancement of mesenchymal cell proliferation by using solubilized amniotic membrane as scaffold' HUM. CELL vol. 21, no. 1, 2008, page A5 + ABSTR. NO. O-02, XP008123513
• KUMAR T.R ET AL.: 'Biocompatible collagen scaffolds from a human amniotic membrane: physicochemical and in vitro culture characteristics' J. BIOMATER. SCI. POLYMER EDN. vol. 14, no. 7, 2003, pages 689 - 706, XP009117131

## Description

Technical Field

The present invention relates to solubilized amnion compositions and uses thereof as a material for matrices for culturing cells.

Background Art

It is known that, when culturing cells on a substrate, cells are cultured on a culture matrix obtained by coating a substrate made of plastic or glass with collagen, fibronectin or the like in order to promote cell growth. It is also known that collagen obtained from an amnion is used as collagen for producing culture matrices (Non-patent Literature 1). It is further known that an amnion *per se* from which cells are removed is used as a culture matrix (Non-patent Literatures 2 and 3).

> Non-patent Literature 1: T. R. KUMAR, et al., J. Biomater. Sci. Polymer Edn. Vol. 14, No. 7, pp. 689-706 (2003)
> Non-patent Literature 2: Morio Ueno et al., PNAS, vol. 103, no.25, 9554-9559, June 20, 2006

Disclosure of the Invention

Problems Which the Invention Tries to Solve

When an amnion *per se* is used as a culture matrix, there are some problems in handling and preservability. That is, cells arc usually cultured in a culture dish or wells of a microplate, and in such cases an amnion has to be cut such that the amnion piece should fit to a culture dish or wells to be used. Furthermore, the amnion piece may be ripped up from the substrate. In particular, in cases where cells are cultured for a long time, it is needed to replace culture medium while keeping cells adhering to the bottom of the culture dish. The amnion gets a physical shock from a liquid flow during the medium replacement to be ripped up from a substrate, and therefore the experiment may be stopped, which is problematic.

Other coating materials such as fibronectin and collagen are usually treated with various enzymes during the production step of the compositions, which enzymes arc in most cases those derived from organisms except human, or those recombinant enzymes obtained by using *E. coli* though they have an amino acid sequence based on the genetic information of human. The possibility that the materials including these enzymes remain in the obtained coating materials raises a serious problem in cases where cells cultured using such coating materials are used in cell transplant therapy or co-cultured with cells to be used in other transplant therapy.

Moreover, other coating materials such as fibronectin and collagen require an enzymatic treatment during the step of preparing products thereof. Purification of enzymes are usually difficult, and in many cases commercial enzymes are expensive. Therefore, there is a problem that preparation of enzymes prior to the preparation step is not easy.

Accordingly, an object of the present invention is to provide a safe, inexpensive material which is better in handling and more suitable for cell culturing than an amnion *per se.*

Means for Solving the Problem

The present inventors arrived at the idea that, if amnions can be solubilized, those prepared by coating a substrate with the solubilization product and drying the coating may be used as a culture matrix. In this case, the culture matrix is good in handling, as the culture matrix can be obtained by applying the solubilization product on a substrate and then drying the product. However, a solubilization product of an amnion has not been known. The present inventors intensively studied to find a method for preparing a solubilized amnion, and find that a substrate coated with the solubilization product of an amnion and then dried can be used as a culture matrix, which is suitable for cell culturing and can be prepared at a low cost as explained in detail below, thereby completing the present invention.

That is, the present invention provides a solubilized amnion composition containing in a medium a solubilization product of an amnion obtained by solubilizing an amnion by acid treatment under heat. The present invention also provides a solubilized amnion coating material obtained by coating a substrate with the above-described composition according to the present invention and drying the composition on the substrate. The present invention further provides a matrix for culturing cells, which is composed of the above-described solubilized amnion coating material according the present invention. The present invention further provides a method of culturing cells, which method comprises culturing cells on the above-described solubilized amnion coating material according to the present invention.

Effects of the Invention

By the present invention, a composition containing a solubilized amnion was first provided. Those obtained by coating a substrate with the composition of the present invention and drying it can be used as a matrix for culturing cells. As the solubilized amnion composition is a liquid, the composition can be prepared as a culture matrix simply by coating a surface of the substrate with the matrix and drying it, and thus is good in handling.

Moreover, the coating material according to the present invention can be produced at a low cost. Cells cultured on the coating material can be used as they are because there is no possibility that materials used during the preparation would remain, and therefore the coating material of the present invention is suitable for the regenerative medicine. Although the solubilized amnion coating material according to the present invention was produced via a step of acid treatment and preferably an additional step of neutralization with a base during preparation of an amnion component, most of the acids or bases used in these steps are volatile, and thus they can be completely removed from the amnion composition by heating. Therefore, cells cultured on the coating material of the present invention which does not contain residues can be used for cell transplantation as they are. Particularly in the field of the regenerative medicine, it is undesirable that cells to be transplanted are subjected to some sort of treatment just before the transplantation, because the cells to be used for cell transplantation will be in many cases differentiated into the objective tissue cells according to a very sophisticatedly designed method for induction of cell differentiation, and also the timing of transplantation may largely affect on whether the therapy will be successful or not. Therefore, the coating material according to the present invention, which makes it possible to transplant the cultured cells as they are, is expected to make a great contribution to the organ reconstruction and the regenerative medicine.

Furthermore, according to the present invention, the composition can be prepared simply using an inexpensive acid arid/or base, and thus the present invention is advantageous in that the coating material can be prepared more simply at a lower cost in comparison with other coating materials.

Brief Description of the Drawings

Fig. 1 shows the results of the evaluation of the cell number when amniotic mesenchymal cells were cultured on various culture matrices in Examples below.

Fig. 2 shows the results of the evaluation of the cell number when human bone marrow mesenchymal stem cells were cultured on culture matrices coated with the human solubilized amnion composition at various concentrations in Examples below.

Fig. 3 shows the results of the evaluation of the cell number when human bone marrow mesenchymal stem cells were cultured on a culture matrix coated with the human solubilized amnion composition in Examples below.

Fig. 4 shows the results of the evaluation of the cell number when human bone marrow mesenchymal stem cells were cultured on various culture matrices in Examples below.

Fig. 5 shows the results of the comparison of the cell yield when human bone marrow mesenchymal stem cells were cultured on various culture matrices and collected by enzymatic treatment in Examples below.

Mode for Carrying out the Invention

As described above, the solubilized amnion composition of the present invention is a composition obtained by treating an amnion with an acid under heat, which composition contains an amnion lysate in a liquid medium.

The animal species from which the amnion is derived is not restricted as long as it is a mammal. In cases where human cells or cells to be transplanted in or administered to human are cultured, human amnion is preferably used as a source material in view of the growth and/or safety of the cultured cells. Amnions are excreted with placentae after delivery and are discarded as medical wastes even in the case of human amnion. Thus, human amnion is preferred because it may also be easily obtained without ethical problems.

It is preferred to remove cells adhering to an amnion and kill the remaining cells prior to the acid treatment. Cells may be removed by repeatedly subjecting an amnion to a vigorous shaking in a buffer. Examples of the treatment for killing the remaining cells include a treatment with a strong alkali or an EDTA solution. As a strong alkali, an alkali metal hydroxide such as sodium hydroxide is preferred. In cases of sodium hydroxide, the preferred concentration is, but not limited to, usually 25 mM to 400 mM, preferably about 50 mM to 200 mM. The concentration of EDTA in EDTA solution is usually 5 mM to 80 mM, preferably about 10 mM to 40 mM. The treatment with a strong alkali or EDTA may be carried out by repeatedly washing the amnion with an strong alkali or EDTA, or by repeatedly subjecting the amnion to a vigorous shaking in an strong alkali or EDTA solution. In cases where such a treatment is carried out, it is preferred to sufficiently wash the amnion with water before the acid treatment in order to remove an strong alkali or EDTA.

The acid used in the acid treatment is preferably an acid which evaporates during drying and thus does not remain in the coating material obtained by coating a substrate with the solubilized amnion composition and drying it, and a solution of acetic acid or hydrogen chloride is preferred. In a case of an acetic acid, its concentration is not limited, and usually about 50 mM to 1 M, preferably about 100 mM to 500 mM.

The amount of the acid used in the acid treatment (the amount of an acid solution when an acid solution is used) is not restricted as long as the amnion can be solubilized, and usually 5 mL to 100 mL, preferably 10 mL to 50 mL per 1 g wet weight of an amnion.

The acid treatment is carried out under heat. The temperature during the acid treatment is not restricted, and usually 80°C to 100°C, preferably 85°C to 95°C. The acid treatment may be carried out till the amnion is completely solubilized, and the treatment time is usually about 60 minutes to 90 minutes.

The composition is preferably neutralized by treatment with a base after the acid treatment. The base is preferably a base which does not remain in the coating material obtained by coating a substrate with the solubilized amnion composition and drying it, and aqueous ammonia is preferred. The concentration of aqueous ammonia is not restricted, and usually 25 mM to 400 mM, preferably about 50 mM to 200 mM. The pH after neutralization is preferably neutral, i.e., pH of about 6.5 to 7.5.

In the manner as described above, the solubilized amnion composition of the present invention which contains a solubilized amnion in a medium may be obtained. The medium is preferably water. By using an aqueous acid solution in the above-described acid treatment, the composition of the present invention wherein the medium is water may be easily obtained. Alternatively, by using an aqueous base solution such as aqueous ammonia in neutralization treatment with a base, the composition of the present invention wherein the medium is water may also be easily obtained.

The solubilized amnion composition of the present invention may be stored in the state of acetic acid solution or in frozen state.

The solubilized amnion coating material may be obtained by coating a substrate with the solubilized amnion composition of the present invention and drying it. Coating may be performed by a well-known method such as application, immersion, spraying or the like. The substrate may be a substrate used in a conventional cell culture, and may be a bottom of a culture dish or a schale, or a bottom of wells of a microtiter plate. The material of the substrate is not restricted, and usually glass or plastics such as polystyrene.

The solubilized amnion coating material of the present invention may be used as a matrix for culturing cells. Although not restricted thereto, in view of cell growth efficiency and safety, the cells to be cultured are preferably human cells or cells to be administered to human if the composition is obtained from a composition of solubilized human amnion. In Examples below, amniotic mesenchymal cells were cultured on the cell culture matrix of the present invention, but the cells are not restricted thereto. Cell culturing *per se* may be carried out in accordance with a common method known for the respective cells (e.g. stem cells).

The coating material according to the present invention can be uniformly applied on a substrate, as coating is carried out by adding an aqueous solution of solubilized amnion to a substrate. Therefore, the matrix is less likely to be ripped up when compared to those using an amnion *per se*, and thus the coating material of the present invention is suitable for a long-term culture. Moreover, the coating material of the present invention has an advantage in that the cells are hardly influenced by culture conditions depending on where they adhere to the culture dish during the culture.

The present invention will now be described more concretely by way of an example thereof. However, the present invention is not restricted to the example below.

Example 1

Preparation of Human Solubilized Amnion Composition (Part 1)

A composition containing a solubilization product of human amnion in water was prepared according to the following procedure.

1) Place 1.8 g (wet weight) of the separated amnion in a 50 mL tube.
2) Add 45 ml of PBS (-) to the amnion, and shake the tube vigorously for 5 minutes at room temperature.
3) Repeat the above another twice. Discard the supernatant.
4) Add 45 ml of 0.1 N NaOH thereto, and shake the tube vigorously for 5 minutes at room temperature. Discard the supernatant.
5) Add 45 ml of 0.1 N NaOH thereto, and shake the tube vigorously for 5 minutes at room temperature. Discard the supernatant.
6) Add 45 ml of 0.1 N NaOH thereto, and shake the tube vigorously overnight at room temperature. Discard the supernatant.
7) Add 45 ml of 0.1 N NaOH thereto, and shake the tube vigorously overnight at room temperature. Discard the supernatant.

8) Add 45 ml of distilled dcionizcd water, and shake the tube vigorously for 5 minutes at room temperature. Discard the supernatant.

9) Repeat the above another 2 times. Discard the supernatant.

10) Add 45 ml of 250 mM acetic acid. Immerse the tube in a 90°C water bath with occasional shaking till the amnion dissolves.

11) Neutralize the resultant with 0.1 M aqueous ammonia.

12) Store die resultant as a solubilized amnion composition.

Example 2

Preparation of Human Solubilized Amnion Composition (Part 2)

A composition containing a solubilization product of human amnion in water was prepared according to the following procedure.

1) Place 1.8 g (wet weight) of the separated amnion in a 50 mL tube.

2) Add 45 ml of PBS (-) to the amnion, and shake the tube vigorously for 5 minutes at room temperature.

3) Wash the amnion 20 times with 45 ml of 0.1 NaOH.

4) Wash the amnion 3 times with 45 ml of distilled deionized water.

5) Add 45 ml of 250 mM acetic acid to the amnion. Immerse the tube in a 90°C water bath with occasional shaking till the amnion dissolves.

6) Neutralize the resultant with 0.1 M aqueous ammonia.

7) Store the resultant as a solubilized amnion composition.

Example 3

Preparation of Human Solubilized Amnion Composition (Part 3)

A composition containing a solubilization product of human amnion in water was prepared according to the following procedure.

1) Place 1.8 g (wet weight) of the separated amnion in a 50 mL tube.

2) Wash the amnion 7 times with 45 ml of 0.02 M EDTA.

3) Wash the amnion 3 times with 45 ml of PBS.

4) Wash the amnion 3 times with 45 ml of distilled deionized water.

5) Add 45 ml of 250 mM acetic acid to the amnion. Immerse the tube in a 90°C water bath with occasional shaking till the amnion dissolves.

6) Neutralize the resultant with 0.1 M aqueous ammonia.

7) Dialyze the mixture against distilled deionized water. Centrifuge it at 3.000 RPM for 3 hours.

8) Store the resultant as a solubilized amnion composition.

Example 4

Use as Culture Matrix (Part 1)

The bottom of a culture dish (Culture area: 0.32 cm$^2$) was coated with the solubilized amnion composition by adding thereto 50 $\mu$l of the human solubilized amnion composition (0.128 mg/mL) prepared in Example 1, and then dried.

Human amniotic mesenchymal cells (about 1,000 cells), which are mesenchymal cells, were seeded onto the culture dish which was coated with the dried solubilized amnion composition, and 100 $\mu$l of DMEM/F12 supplemented with 10% FBS was added thereto as a culture medium, followed by culturing the cells at 37°C. For comparison, human amniotic mesenchymal cells were cultured under the same condition as described above except that commercially available culture dishes coated with type I collagen, fibronectin, laminin or HEM (human extracellular matrix) were used. For control, the cells were also cultured under the condition that they were seeded directly onto a culture dish or seeded onto a culture dish to which 100 $\mu$l of distilled deionized water was added and then dried. A reagent (reagent for measurement of cell growth) was directly added to the culture dishes 3. 6 and 8 days after the start of the culture, and the absorbance was measured to assess the number of the cells.

The culture dishes coated with the each matrices used for comparison were prepared as follows.

(1) A commercially-available 96-well plate coated with type 1 collagen (4860-010, Iwaki) was used as a type I collagen-coated culture dish.

(2) As for fibronectin, 50 $\mu$l of a solution prepared by diluting a commercial fibronectin (Iwaki, 02-070-002) to a final concentration of 10 $\mu$g/mL with phosphate buffered saline (PBS) was added to each wells to coat the bottoms of a 96-well culture dish (FALCON), and then the wells were dried overnight at 37°C, followed by washing the wells twice with PBS to obtain a fibronectin-coated culture dish.

(3) As for laminin, 50 $\mu$l of a solution prepared by diluting a commercial laminin (Upstate, 08-125) to a final concentration of 34 $\mu$g/mL with PBS was added to each wells to coat the bottoms of a 96-wcll culture dish (FALCON), and then the wells were dried overnight at 37°C, followed by washing the wells twice with PBS to obtain a laminin-coated culture dish.

(4) As for HEM, 50 $\mu$l of a solution prepared by diluting a commercial HEM (BD Biosciences, 354237) to a final concentration of 20 $\mu$g/mL with PBS was added to each wells to coat the bottoms of a 96-well culture dish (FALCON), and then the wells were dried overnight at 37°C, followed by washing the wells twice with PBS to obtain a HEM-coated culture dish.

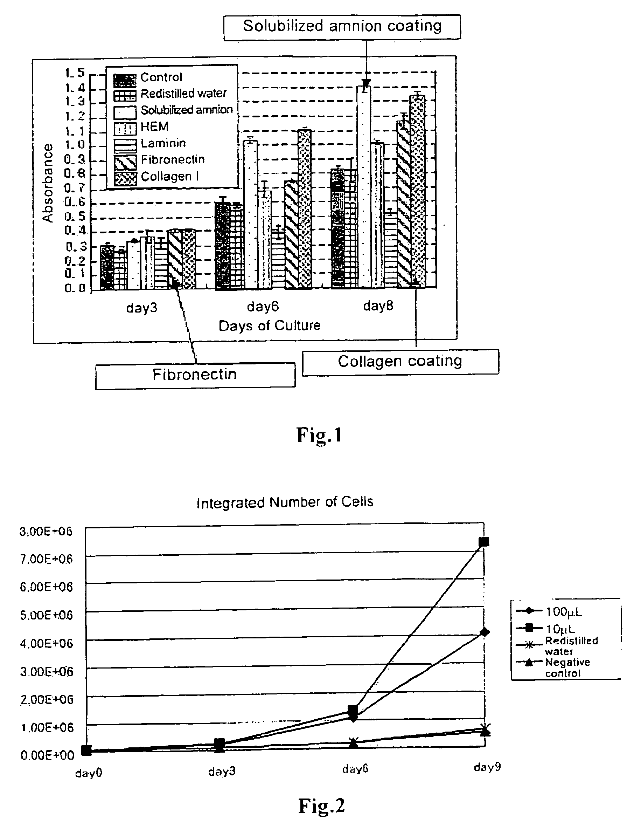

The results are shown in Fig. 1. As shown in Fig. 1, the cell number was the highest and thus the cell growth was promoted most effectively when mesenchymal cells were cultured on the culture dish coated with the dried solubilized amnion composition of the present invention.

Example 5

Use as Culture Matrix (Part 2)

An aqueous solution of the human solubilized amnion composition prepared by adding 10 $\mu$l or 100 $\mu$l of the human solubilized amnion composition (0.128 mg/mL) to 2 ml of distilled water was added to a culture dish with culture area of 9.6 cm$^2$ (35-1008; FALCON) to coat the bottom of the culture dish with the solubilized amnion composition, and then the dish was dried.

Human mesenchymal stem cells (hMSCs) (about 50,000 cells) derived from the bone marrow, which were tissue stem cells, were seeded onto the culture dish coated with the dried solubilized amnion composition, and 100 $\mu$l of DMEM/F12 supplemented with 10% FBS was added thereto as a culture medium, followed by culturing the cells at 37°C. For comparison, the cells were also cultured by seeding directly onto a culture dish (NC: Negative Control) or onto the bottom of a culture dish to which 100 $\mu$l of distilled deionized water was added and then dried (DW: Distilled Water). A reagent (reagent for measurement of cell growth) was directly added to the culture dishes 3, 6 and 9 days after the start of the culture, and the absorbance was measured to assess the cell number. In addition, the cell number was also assessed in the same manner 5, 10 and 15 days after the start of the culture.

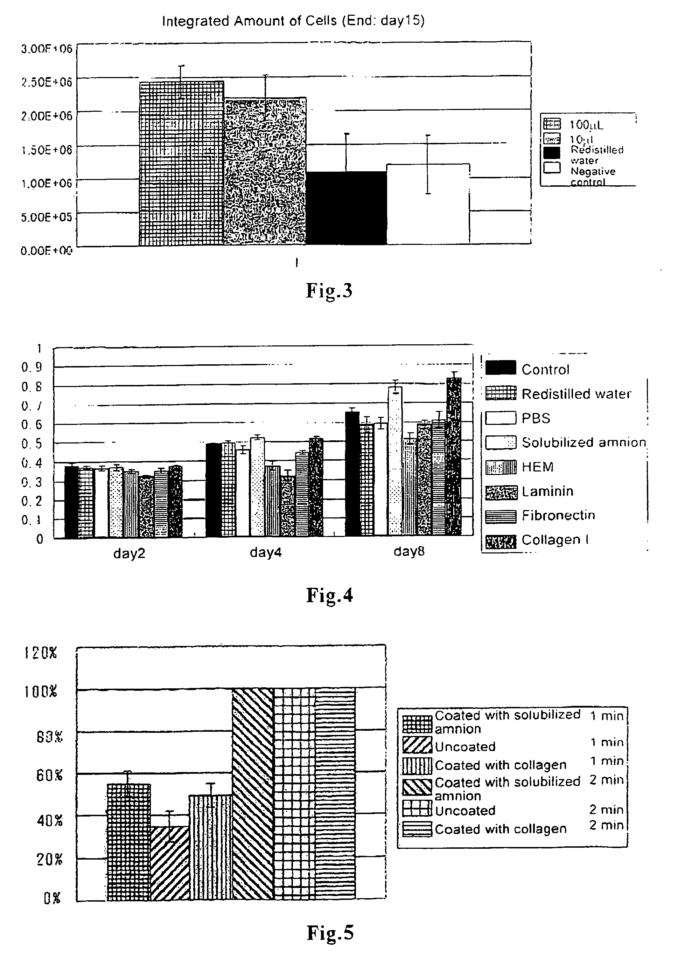

The results are shown in Figs 2 and 3. As shown in Fig. 2, hMSC cells grew in a manner depending on the concentration of the solubilized amnion composition when the cells were cultured on the culture dish coated with the dried solubilized amnion composition of the present invention.

As shown in Fig. 3, an effect to significantly proliferate hMSC cells can be obtained when the cells were cultured on the culture dish coated with the dried solubilized amnion composition of the present invention.

Example 6

Use as Culture Matrix (Part 3)

The bottoms of a 96-well culture dish (FALCON) were coated with the solubilized amnion composition by adding 50 $\mu$l of the human solubilized amnion composition prepared in Example 1 to the culture dish with culture area of 0.32 cm$^2$ and then the culture dish was dried.

Onto the culture dish coated with the dried solubilized amnion composition, hMSCs (about 1,000 cells) were seeded, and 100 $\mu$l of DMEM/F12 supplemented with 10% FBS was added as a culture medium, followed by culturing the cells at 37°C. For comparison, hMSCs were cultured under the same condition as described above except that commercial culture dishes coated with type I collagen, fibronectin, laminin or HEM (human extracellular matrix) were used.

The culture dishes coated with each matrices used for comparison were prepared in the same manner as in Example 4 above.

For further comparison, the cells were also cultured under the condition that they were seeded directly onto a culture dish or seeded onto a culture dish to which 100 $\mu$l of distilled deionized water was added and then dried. A reagent (reagent for measurement of cell growth) was directly added to the culture dishes 2, 4 and 8 days after the start of the culture, and the absorbance was measured to assess the cell number.

The results are shown in Fig. 4. As shown in Fig. 4, the cell number was the highest and thus the cell growth was promoted most effectively when hMSCs were cultured on the culture dish coated with the dried solubilized amnion composition of the present invention.

Example 7

Study on Adhesion Ratio of Mesenchymal Stem Cells

To each of 3.0 cm Petri dishes (FALCON, 3001), 0.1 ml of the human solubilized amnion composition. (0.128 mg/mL) was added to coat the bottom thereof with the amnion composition, and the dishes were dried.

Onto the culture dish coated with the dried solubilized amnion composition. hMSCs (about $2.0 \times 10^5$ cells) were seeded, and 3.0 ml of MSCBM (Mesenchymal Stem Cell Basal Medium) (Catalog No.: CAMBRE, obtained from Takara, PT-3001) was added thereto, followed by culturing the cells at 37°C. For comparison, the cells were cultured under the same condition as described above except that a commercially-available 100 mm Type J Collagen-Coated Dish (Iwaki, 25020 COL1) or an uncoated dish was used. Within 24 hours after the start of the culture, 0.1 ml of 0.1% trypsin-EDTA was added, and the dish was gently shaken in an incubator at 37°C. The enzymatic reaction was terminated 1 minute or 2 minutes after the addition of trypsin-EDTA by adding a culture medium. Floating cells were collected and the number of the cells was counted. The same experiment was repeated 3 times for each line to calculate the standard deviation of the floating cell number. Cell yield was calculated according to the equation below.

$$\text{Yield (\%)} = (\text{Number of collected floating cells} \times 100) / (\text{Total cell number})$$

The results are shown in Fig. 5. The yield of hMSC cultured on a solubilized amnion-coated dish, collagen-coated dish, and uncoated dish was $55 \pm 6\%$, $49 \pm 6\%$, and $35 \pm 8\%$, respectively. In the case of a solubilized amnion-coated dish, the cultured cells were more easily separated, which was significantly different from an uncoated dish. These results indicate that, when a culture dish coated with the solubilized amnion is used, cells can be collected with a high efficiency by trypsin treatment for a short time, and thus an excellent technical effect to decrease damages to the cultured cells caused by the enzyme activity of trypsin can be obtained.

On the other hand, Fig. 5 shows no significant difference in the cell yield between a solubilized amnion-coated dish and a collagen-coated dish. However, since various enzymes arc used for purifying collagen, collagen-coated dishes have the problem that the materials used in its purification may remain. Therefore, it is now considered to be dangerous to transplant cells cultured in presence of collagen in a body as they are. In contrast, in the case of the solubilized amnion-coated dish, acetic acid and ammonia used in solubilization of an amnion can be completely removed by volatilization. Thus, cells cultured on a culture dish coated with the solubilized amnion may be used in cell transplantation as they are while avoiding the risk associated with the remaining materials used in the purification, which is an excellent technical feature.

Example 8

Components of Solubilized Amnion Composition

Components contained in the solubilized amnion composition prepared in Example 1 were analyzed. As a result, the main components were as follows.

    1. Protein Concentration: 60 to $300\mu$g/ml
    2. Lipid Analysis: Phospholipids, glycolipids and ganglioside are contained.
    3. Collagen: Found.
    4. Laminin: Found in an amnion before solubilization (by immunostaining).

The results above revealed that the solubilized amnion composition contains not only collagen but also other components contained in biomembrane, and its composition is similar to that of biomembrane.

**Claims**

1. A solubilized amnion composition comprising in a medium a solubilization product of an amnion obtained by solubilizing an amnion by a treatment with acetic acid or hydrogen chloride under heat, wherein the pH of the composition is neutral by neutralization with aqueous ammonia.

2. The composition according to claim 1, wherein said medium is water.

3. The composition according to any one of claims 1 to 2, wherein said amnion is of human origin.

4. A solubilized amnion coating material obtained by coating a substrate with the composition according to any one of claims 1 to 3 and drying the composition on the substrate.

5. A matrix for culturing cells, which is composed of the solubilized amnion coating material according to claim 4.

6. The matrix according to claim 5, wherein said cells are mesenchymal cells.

7. The matrix according to claim 6, wherein said mesenchymal cells are derived from human amnion.

8. The matrix according to claim 5, wherein said cells are mesenchymal stem cells.

9. The matrix according to claim 8, wherein said mesenchymal stem cells are derived from human bone marrow.

10. A method of culturing cells, which method comprises culturing cells on the solubilized amnion coating material according to claim 4.


**Patentansprüche**

1. Löslich gemachte Amnionzusammensetzung, die in einem Medium ein Solubilisierungsprodukt eines Amnions aufweist, das durch Löslichmachen eines Amnions durch Behandlung mit Essigsäure oder Chlorwasserstoff unter Wärme erhalten wurde, wobei der pH-Wert der Zusammensetzung durch Neutralisieren mit wäßrigem Ammoniak neutral ist.

2. Zusammensetzung nach Anspruch 1, wobei das Medium Wasser ist.

3. Zusammensetzung nach einem der Ansprüche 1 bis 2, wobei das Amnion humanen Ursprungs ist.

4. Löslich gemachtes Amnion-Beschichtungsmaterial, das durch Beschichten eines Substrats mit der Zusammensetzung nach einem der Ansprüche 1 bis 3 und Trocknen der Zusammensetzung auf dem Substrat erhalten wird.

5. Matrix zum Züchten von Zellen, die aus dem löslich gemachten Amnion-Beschichtungsmaterial nach Anspruch 4 besteht.

6. Matrix nach Anspruch 5, wobei die Zellen Mesenchymzellen sind.

7. Matrix nach Anspruch 6, wobei die Mesenchymzellen von humanem Amnion stammen.

8. Matrix nach Anspruch 5, wobei die Zellen Mesenchym-Stammzellen sind.

9. Matrix nach Anspruch 8, wobei die Mesenchym-Stammzellen von humanem Knochenmark stammen.

10. Verfahren zum Züchten von Zellen, wobei das Verfahren das Züchten von Zellen auf dem löslich gemachten Amnion-Beschichtungsmaterial nach Anspruch 4 aufweist.

**EP 2 149 601 B1**

**Revendications**

1. Composition d'amnios solubilisé comprenant dans un milieu un produit de solubilisation d'un amnios obtenu en solubilisant un amnios au moyen d'un traitement à l'acide acétique ou au chlorure d'hydrogène sous chaleur, dans lequel le pH de la composition est neutre par neutralisation avec de l'ammoniac en solution aqueuse.

2. Composition selon la revendication 1, dans laquelle ledit milieu est de l'eau.

3. Composition selon l'une quelconque des revendications 1 à 2, dans laquelle ledit amnios est d'origine humaine.

4. Matériau de revêtement d'amnios solubilisé obtenu en revêtant un substrat avec la composition selon l'une quelconque des revendications 1 à 3 et en séchant la composition sur le substrat.

5. Matrice pour cultiver des cellules, qui est composée du matériau de revêtement d'amnios solubilisé selon la revendication 4.

6. Matrice selon la revendication 5, dans laquelle lesdites cellules sont des cellules mésenchymateuses.

7. Matrice selon la revendication 6, dans laquelle lesdites cellules mésenchymateuses sont dérivées d'amnios humain.

8. Matrice selon la revendication 5, dans laquelle lesdites cellules sont des cellules souches mésenchymateuses.

9. Matrice selon la revendication 8, dans laquelle lesdites cellules souches mésenchymateuses sont dérivées de moelle osseuse humaine.

10. Procédé de culture de cellules, lequel procédé comprend la culture de cellules sur le matériau de revêtement d'amnios solubilisé selon la revendication 4.

Fig.1

Fig.2

Integrated Amount of Cells (End: day15)

**Fig.3**

**Fig.4**

**Fig.5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. R. KUMAR et al.** *J. Biomater. Sci. Polymer Edn.,* 2003, vol. 14 (7), 689-706

- **MORIO UENO et al.** *PNAS,* 20 June 2006, vol. 103 (25), 9554-9559